# EUROPEAN PATENT APPLICATION

(11) **EP 1 872 818 A1**
(43) Date of publication of application: **02.01.2008**
(21) Application number: 06253165.2
(22) Date of filing: 20.06.2006
(51) Int. Cl.: A61M 21/00, G08B 21/02

(54) **Electronic baby-soothing device**

(71) Applicant: Future Acoustic LLP, London SW7 2EU (GB)
(72) Inventor: Raptopoulos, Andreas, London SE1 9LF (GB); Barnado, Christopher John Andrew, Hertfordshire CM23 5JP (GB)
(74) Representative: Addison, Ann Bridget

(57) **Abstract**

The present invention provides a baby soother comprising a transducer (12) for detecting crying by the baby and for generating electrical signals representing the crying sound and a processor (16, 30, 36) for analysing the electrical signals to evaluate the crying and provide a control output. A sound bank (44) stores audio data representing soothing sounds, and sound generating means (42) responsive to the control output serves for selecting audio data from the sound bank and providing an audio output, which is ouput by output means (24) as sound.

## Description

This invention concerns a baby soother for use in a baby's room for monitoring the baby's voice and for generating a calming or soothing sound output in the event of the baby crying.

Baby monitors are known comprising a transmitter in the baby's room for transmitting signals representing the noises produced by the baby to another room, and a receiver in the other room for providing a corresponding output either on a display or by way of a loudspeaker. This enables a parent or other carer to monitor the state of the baby and to tend to the baby in the event of distress.

Sound screening systems are also known for monitoring and regulating sound in an environmental space, such systems detecting the ambient noise and generating masking sound or tonal sequences to generate pleasing sound in the environmental space. In particular, sound screening systems are described in earlier published International Patent Applications Nos. WO 01/37256 and WO 02/25631 and published US Patent Application No. 10/996330, all of which are incorporated herein by reference.

However, none of these known arrangements is designed to monitor a baby's state and then address this state directly should the baby be crying.

It is an aim of the present invention to provide a baby soother for monitoring crying by a baby and for generating a soothing sound in response to crying.

It is another aim of the present invention to provide a device for detecting and monitoring a baby's crying, for evaluating the baby's crying and for generating a soothing sound in dependence upon the outcome of the evaluation.

According to one aspect of the present invention, there is provided a baby soother comprising a transducer for detecting crying by the baby and for generating electrical signals representing the crying sound, a processor for analysing the electrical signals to evaluate the crying and provide a control output, a sound bank storing audio data representing soothing sounds, sound generating means responsive to the control output for selecting audio data from the sound bank and providing an audio output, and output means for outputting the audio output as sound.

According to another aspect of the present invention, there is also provided a method of monitoring the crying by a baby comprising detecting crying by the baby and generating electrical signals representing the crying sound; analysing the electrical signals to evaluate the crying; providing a control output based on the evaluation; storing audio data representing soothing sounds; in response to the control output selecting audio data and providing an audio output; and outputting the audio output as sound.

The processor may analyse the electrical signals in order to monitor one or more of the crying pattern, the character of the crying, and changes in the crying.

Advantageously, the sound bank stores a selection of pre-recorded tonal and atonal sound samples and/or a selection of user recorded sound samples.

In a preferred embodiment of the invention the baby soother is incorporated in a baby listener including a transmitter device for placing in the baby's room and a receiver device for placing in a room occupied by a parent or other carer.

The invention will be described further, by way of example, with reference to the accompanying drawings, in which:
Figure 1 is a schematic block diagram representing a baby soother according to the present invention;
Figure 2 is a more detailed block diagram of the baby soother of Figure 1;
Figure 3 is a software flowchart representing the steps taken by a processor of the baby soother of Figures 1 and 2 to monitor and respond to crying by the baby;
Figure 4 is a signal diagram showing signals representing crying sound energy as monitored by the soother, processed crying sound energy and output sound in different output modes of the soother;
Figure 5 is a block diagram representing an application of the baby soother of Figures 1 to 4 in a first embodiment of baby listening device; and
Figure 6 is a block diagram representing an application of the baby soother of Figures 1 to 4 in a second embodiment of baby listening device.

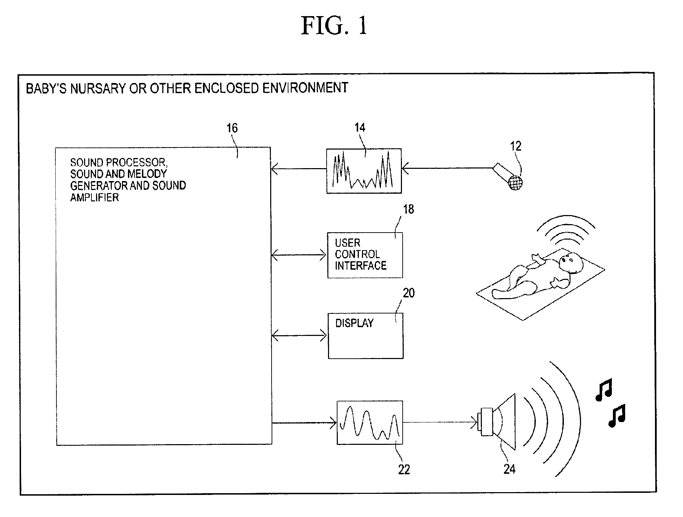

Referring initially to Figures 1 to 4, a baby soother according to the invention comprises a portable device 10 for use in the baby's nursery or other room.

The device 10 has a microphone 12 for picking up sounds produced by the baby and for generating an analog audio signal represented in box 14 for supply to a sound processing unit 16. The sound processing unit 16 is controlled by means of a user control interface 18 operable, by means of manual knobs, dials, keys, proximity, voice or other conventional control means, to enable the user to set a functional state or control other settings of the sound processing unit 16. Such user set functional state and settings are indicated on a display 20, for example a light emitting diode display or a liquid crystal display. In dependence upon the set functional state and the nature and volume of the baby's sounds, the sound processing unit 16 generates an analog output signal represented in box 22 for supply to a speaker 24 for output.

The baby soother as illustrated in Figure 1 is designed to detect when the baby cries, to distinguish between different types and levels of crying, and to produce soothing sounds to calm the baby and/or to encourage a sleeping state in the event of crying.

In a preferred embodiment as described below, the sound processing unit 16 incorporates a logic control circuit and software enabling the soother to distinguish between non-crying and crying sounds and between different kinds of crying. The sound processing unit 16 is capable of generating a range of discrete and blended pre-recorded tonal and atonal sounds and user recorded sounds at a range of volumes depending on whether the baby is crying, how loud the baby is crying, and on what type of crying noises the baby is making.

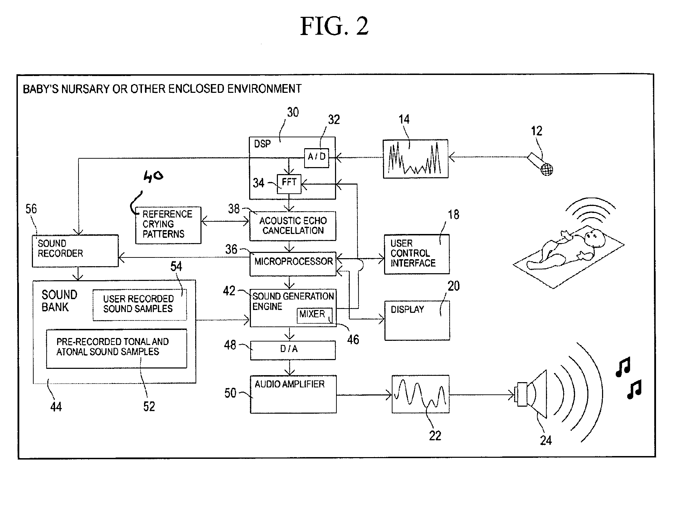

The sound processing unit 16 will now be described further with reference to Figure 2.

As shown in Figure 2, the sound processing unit 16 comprises a digital signal processor (DSP) 30 for receiving the analog audio signal 14 from the microphone 12. The DSP 30 comprises an analog to digital converter 32, which receives the analog audio signal 14 and generates a data analysis output as output. The DSP 30 also comprises a fast fourier transform (FFT) analysis circuit 34 arranged to receive the data analysis output in order to analyse and divide the input sound energy into a number of critical frequency bands for further analysis by a microprocessor 36. These frequency bands can be set to coincide with the 25 critical bands of human hearing or can be set differently as may be suitable for detecting the prominent characteristics of the crying sounds.

The data analysis output from the DSP 30 provided by the FFT circuit 34 is then supplied by way of an acoustic echo cancellation circuit 38 (to be described below) to the microprocessor 36, which is also connected to the user control interface 18 and display 20. The microprocessor 36 is further connected by way of the acoustic echo cancellation circuit 38 to a reference bank 40 storing data representing typical crying patterns and, in response to receipt of the data analysis output from the DSP 30, the microprocessor 36 compares the data analysis output with the data in the reference bank 40 to establish whether the output corresponds with a respective crying pattern and therefore represents a particular kind of crying. Subject to the outcome of the comparison, the microprocessor 36 generates a corresponding control output. For example, the reference bank 40 may contain pre-recorded data patterns for ordinary crying and for particular types of crying that represent pain or hunger, and the microprocessor 36 may generate a first control output in the case of ordinary crying and a second control output in the case of crying representing pain or hunger.

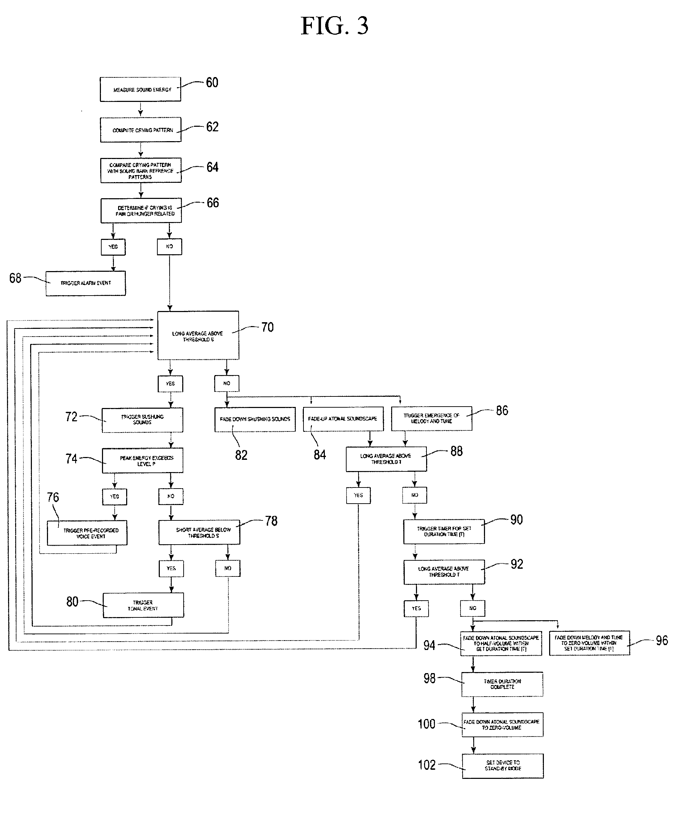

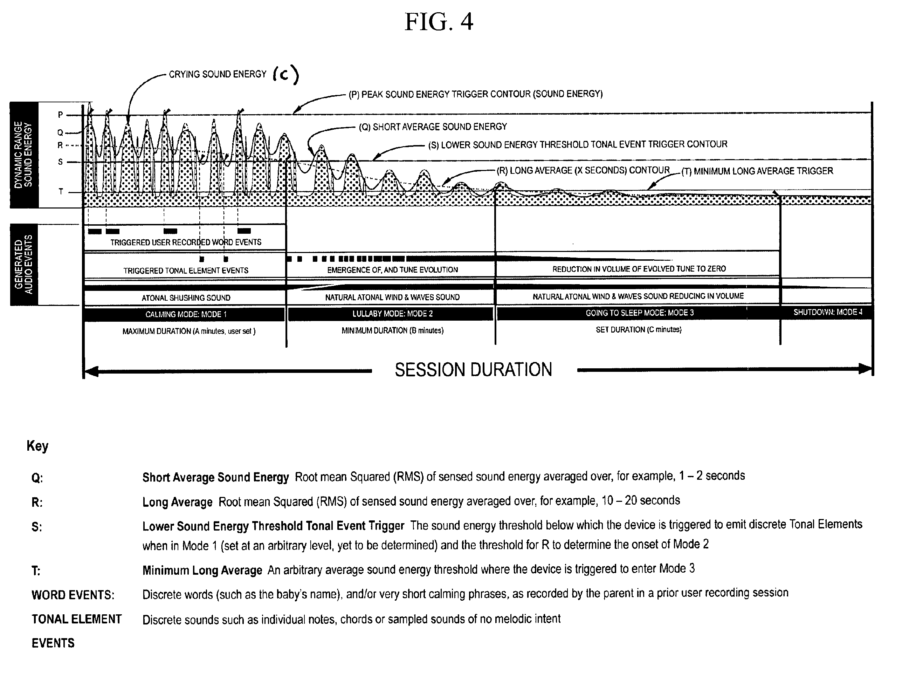

The microprocessor 36 further analyses the data analysis output from the DSP 30 in order to detect certain elements in the sounds produced by the baby and to generate a corresponding control output and trigger certain output sound modes in dependence upon such detection, as described below in relation to Figures 3 and 4.

The control output from the microprocessor 36 is supplied to a sound generation engine 42, which responds by generating a digital audio output representing a particular type of sound and which sets an output volume. For this purpose, the sound generation engine 42 refers to a sound bank 44 containing sound samples and selects at least one of the sound samples for the sound output. In the preferred embodiment of the invention shown in Figure 2, the sound generation engine 42 includes a mixer circuit 46 for mixing and blending a selected combination of the sound samples.

The digital output from the sound generation engine 42 is supplied to a digital to analog converter 48 whose analog output is amplified in an audio amplifier 50 for supply to the speaker 24.

User adjustment of the manual controls on the user control interface 18 enables the control of various characteristics of the sound output by the speaker 24 such as volume, type and range of sound, output mode, duration of output mode etc.

Further, in order to eliminate acoustic echo generated as a result of the microphone 12 detecting sound output by the speaker 24, a feedback connection is provided from the sound generation engine 42 to the DSP 30 and by way of the acoustic echo cancellation circuit 38 to the microprocessor 36.

The sound samples stored in the sound bank 44 may be pre-recorded and/or user recorded, and in the present embodiment the sound bank 44 comprises a first store 52 of pre-recorded tonal and atonal sound samples stored in the sound bank 44 at point of manufacture, and a second store 54 of user recorded sound samples recorded to the sound bank 44 by the user, following purchase of the baby soother.

For the purpose of recording the user recorded sound samples 54 in sound bank 44, the DSP 30 is connected to the sound bank 44 by way of a sound recorder 56, and the user control interface 18 includes a control setting for setting the sound processing unit 16 into a recording mode. In this mode, a parent of the baby, or other familiar figure, may record specific sounds, for example familiar name(s), familiar word(s), short calming phrases, or simply murmuring, in their own voice by way of the microphone 12. Such sounds are recorded and stored in the sound bank 44 for future use.

Referring now to Figures 3 and 4, an example of a particular monitoring session will be described.

Referring to Figure 4, the analog electrical signal from the microphone 12 is represented by the shaded area (C) representing the sound energy of the noise made by the baby, in this instance crying. The vertical axis of the shaded area represents the amplitude of the sound energy or the level of crying, and the horizontal axis represents time duration.

This sound energy (C) is received in the microprocessor 36 and in step 60 in Figure 3 is measured by tracking the signal over time in the range of frequency bands set in the FFT circuit 34. Next, in step 62, the microprocessor computes the crying pattern. This involves tracking specific characteristics of the sound energy C, for example low energy points representing the onset and ending of crying, peak levels of crying, the attack and decay of the crying sound, and the start and stop of inspiration (intake of breath) during crying. The microprocessor 36 specifically calculates the following:

Firstly, the microprocessor 36 makes near real time measurements of the sound energy C. Secondly, the microprocessor 36 calculates average values for the sound energy C, to produce creating two average values as shown in Figure 4: A short average, based on the root mean squared (RMS) sound energy of the sensed sound averaged over a shorter time period, of for example 1 to 2 seconds, and a long average based on the RMS sound energy of the sensed sound averaged over a longer time period, of for example 10 to 20 seconds. As shown in Figure 4, the short average is a continually oscillating value sensitive to the peaks and troughs of the sound energy C but with some degree of smoothing such that short periods of quiet can be determined, while the long average R tracks overall crying volume over time and may be used to determine whether or not the baby is generally quietening down. In Figure 4, the long average R is shown as a gradually decaying value, because in the example given the crying is gradually diminishing and dies away. In the event that the baby should start crying louder, however, the long average R may be used to determine whether the microprocessor 36 should revert to a previous mode.

Next, in step 64, the microprocessor 36 compares the crying pattern with the data representing crying patterns in the reference bank 40 seeking the closest match for determining the type of crying that is in progress. If the crying is considered to represent pain or hunger, the microprocessor 36 proceeds in step 68 to generate an alarm output. Otherwise, the microprocessor 36 proceeds to step 70 as described below.

In step 70, the microprocessor 36 takes the long average value R and compares it with a lower sound energy threshold S. If R is above the threshold S, the microprocessor 36 enters a first mode of sound output referred to as a calming mode and performs a series of steps 72 to 80, as follows.

For the whole of the duration of the calming output, the control output of the microprocessor 36 in step 72 causes the sound generation engine 42 to output an atonal sound sample in the form of a shushing sound from the prerecording sound samples 52. The microprocessor 37 proceeds to step 74 and compares the peak amplitudes of the sound energy C with another threshold value P constituting a peak threshold. The control output of the microprocessor 36 triggers the output of user recorded voice samples as individual events on each occasion that the peak value of the sound energy C exceeds the peak threshold P, as shown in step 76. Following this step, the microprocessor reverts to step 70 to check the relationship of the long average value R to the lower sound energy threshold S again.

If the peak amplitude value of the sound energy C remains below the peak threshold P, as detected in step 74, the microprocessor 36 proceeds to step 78 and compares the short average value Q with the lower sound energy threshold S. In the event that the troughs of the short average Q drop below the lower sound energy threshold S, the microprocessor 36 causes the sound generation engine 42 in step 80 to trigger tonal sound events such as notes or chords each time that the threshold S is crossed. The microprocessor 36 then reverts to step 70. In the event that the short average Q is not below the threshold S, the microprocessor 36 reverts directly to step 70.

When the long average R crosses the lower sound energy threshold S, as determined in step 70, the microprocessor 36 issues a control output in steps 82 to 86 to cause the sound generation engine 42 to shift from the first mode of sound output to a second mode of sound output. At this point, the microprocessor 36 causes the shushing sounds to begin to fade down in step 82 and the atonal sounds to begin to fade up in step 84, and triggers the emergence of a melody or tune in step 86.

The microprocessor 36 monitors the long average R in relation to a further threshold T, designated the minimum long average, in step 88, reverting to step 70 so long as the long average R remains above the threshold T. At the point where the long average R crosses below the minimum long average threshold T, the microprocessor 36 issues a control output in step 90 causing the sound generation engine 42 to move from the second mode of sound output to a third mode of sound output and set a timer for a predetermined period. The microprocessor 36 then checks in step 92 whether the long average value R is above the threshold T, and if so reverts to step 70. If not, however, the microprocessor 36 issues another control output in steps 94 and 96 causing the sound generation engine 42 to shift from the third mode of sound output to a fourth mode of sound output. At this point, the microprocessor 36 causes the atonal sounds to fade to half the current volume in step 94, and the melody to reduce to zero in step 96.

When the predetermined time period, as set in step 90 and determined in step 98, is complete, the microprocessor in step 100 issues a control output to cause the sound generation engine 42 to fade the atonal sounds to zero volume, and then in step 102 sets the soother into stand-by mode.

Accordingly, referring now particularly to Figure 4, in the first mode of sound output referred to as the calming mode, the control output of the microprocessor 36 triggers an atonal shushing sound. The control output of the microprocessor 36 also triggers the output of a tonal sample from the pre-recorded sound samples 52 in the sound bank 44 on each occasion that the short average Q drops below the lower sound energy threshold S, and these constitute tonal element events. As can be seen, the output of these different sounds has a calming effect on the baby and gradually the sound energy (C) reduces bringing the peaks below the peak threshold P and the long average R below the threshold S. This triggers a transition from the first mode of sound output, the calming mode, to the second mode of sound output, referred to as a lullaby mode.

In the lullaby mode, the user voice samples are no longer emitted, since the peak energy of the crying is below the threshold P. When the short average Q is below the lower sound energy threshold S, tonal sound is emitted as part of a melody or tune generated by the sound generation engine 42 from the pre-recorded sound samples 52, until complete melodies emerge and evolve. At the same time, the atonal shushing sound from the pre-recorded sound samples 52 is replaced by alternative atonal sounds, resembling sounds of wind or waves, or other natural or artificial soothing sounds. The duration of the second mode of sound output, the lullaby mode, continues until the long average R drops below the minimum long average threshold P, at which point the control output of the microprocessor 36 causes the sound generation engine 42 to move into a third mode of sound output, referred to as the going to sleep mode.

The going to sleep mode continues for a predetermined set time controlled by the control output of the microprocessor 36, and during the going to sleep mode, the control output of the microprocessor 36 causes the sound generation engine 42 gradually to reduce the volume of the melody so that after half the set time, the volume of tonal melodies and tunes are reduced to zero. During the going to sleep mode, the atonal sound from the pre-recorded sound samples 52 are also reduced in volume in proportion to the reduction in sound energy coming from the baby as measured by the long average. At the end of the set time period for the going to sleep mode, the microprocessor 36 causes the sound generation engine 42 to move to the forth mode of sound output, in which the overall sound output is gradually shut down. In all of this, the volume of the output sound is held proportionate to the amplitude of the long average R of the sound energy C.

Thus, in the calming mode, the soother outputs voice events in a familiar voice recorded by a parent or other family member as well as atonal shushing sounds, which might for example be short pulses of 200ms duration of broadband "whitenoise" repeated 2 to 4 times per second. The voice events are arranged to coincide with peaks in the crying to catch the baby's attention. In addition, tonal sounds, such as discrete notes or chords, are arranged to be generated to coincide with intervals in the crying represented by the short average Q when below the lower sound energy threshold S, so as to reward the baby for quieting and promote a decrease in crying and an increase in quieter moments. In the lullaby mode, the shushing sounds are reduced in periodicity and/or volume and are blended and gradually replaced by gently modulating atonal sounds, such as those of the wind or waves. The discrete tonal events are replaced by melodic sounds which coalesce and evolve to become part of a recognisable melody or lullaby. In the going to sleep mode, the melody is gradually phased out and its volume reduced to zero and the sound of the wind and waves is also reduced in volume, to be reduced to zero in the shutdown mode when the soother enters a silent, standby state.

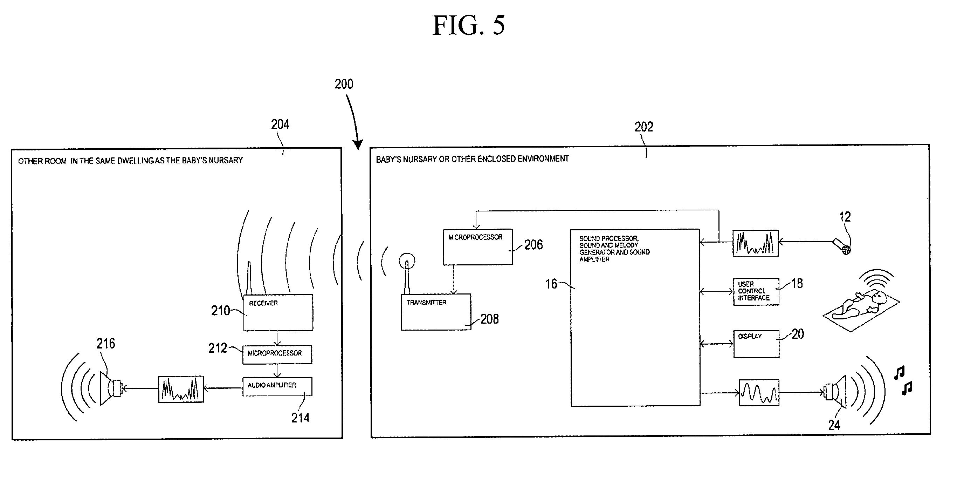

Turning now to Figure 5, an application of the baby soother of Figures 1 to 4 in a baby listener 200 including a transmitter unit 202 and a receiver unit 204 will be described. The transmitter unit 202 is designed for placing in the baby's room for listening to the sounds produced by the baby and for transmitting a wireless communication representing the sounds to the receiver unit 204 in another room occupied by a parent or other carer. Accordingly, the transmitter unit 202 comprises all of the features of the Figure 1 soother and the same elements are designated by the same reference numerals and will not be described further. In addition, the transmitter unit 202 comprises a second microprocessor 206 for processing the analog audio signal 14 from the microphone 12 and supplying an output to a transmitter 208. The transmitter 208 communicates wirelessly with a receiver 210 in the receiver unit 204, which supplies an output to a receiver microprocessor 212 for processing the received signals. The microprocessor 212 supplies an output representing the sounds from the baby to an amplifier 214 and thence to a loudspeaker 216 for alerting the parent or carer to the current state of the baby.

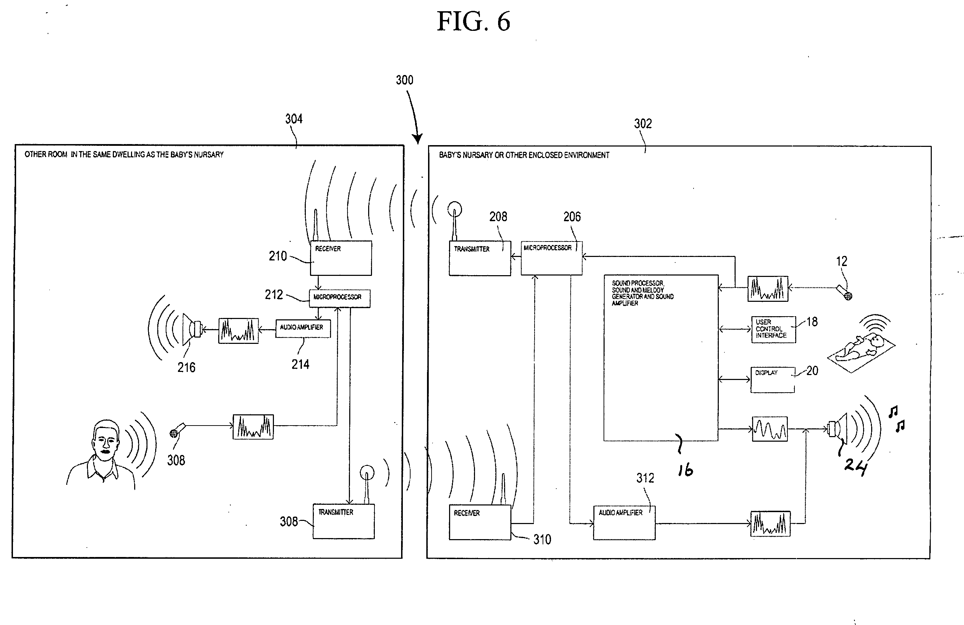

In a modification of the baby listener as illustrated in Figure 6, the invention is incorporated in a two-way baby listener 300, which enables the parent or other carer not only to listen to the baby but also to talk to and communicate with the baby.

The baby listener 300 comprises a baby unit 302 for placing in the baby's room and a carer unit 304 for placing in a room occupied by the parent or other carer. Both units comprise all the features of the Figure 5 baby listener 200, and the same elements are designated by the same reference numerals and will not be described further. In addition, however, the carer unit 304 comprises a microphone 306 for conveying an analog audio signal produced by the parent or carer to the microprocessor 212 for processing. An output from the microprocessor 212 is supplied to a transmitter 308 in the carer unit 304 for transmitting a wireless communication to a receiver 310 in the baby unit 302. An output from the receiver 310 is supplied to the microprocessor 206 for processing and an analog audio output from the microprocessor 206 is supplied to an audio amplifier 312 and thence to the speaker 24 for output. This allows the parent or carer to communicate directly with the baby and to talk to them or sooth them without needing to enter their room.

It will be appreciated that various modifications are possible, within the scope of the invention, to the embodiments described above.

For example, the baby soother can easily be modified to output algorithmically generated sounds, such as lullabies or other gently responsive tonal phrases, to create a sensory rich environment comprising sound evolving on the basis of prescribed tonal evolution rules.

## Claims

1. A baby soother comprising:
a transducer for detecting crying by the baby and for generating electrical signals representing the crying sound;
a processor for analysing the electrical signals to evaluate the crying and provide a control output;
a sound bank storing audio data representing soothing sounds;
sound generating means responsive to the control output for selecting audio data from the sound bank and providing an audio output; and
output means for outputting the audio output as sound.

2. A baby soother according to claim 1 comprising a transmitter device including at least the transducer and a transmitter, and a receiver device comprising a receiver and further output means.

3. A baby soother according to claim 2 in which the transmitter device further includes a receiver, and the receiver device further includes a transmitter.

4. A baby soother according to claim 1, 2 or 3 in which the processor analyses the electrical signals into frequency bands for monitoring a crying pattern.

5. A baby soother according to any preceding claim in which the processor analyses the electrical signals to monitor changes in crying.

6. A baby soother according to any preceding claim in which the processor analyses the electrical signals to detect at least one of a peak value and an average value.

7. A baby soother according to claim 6 in which the processor provides a control output dependent upon a relationship between the value detected and a predetermined threshold.

8. A baby soother according to any preceding claim in which the sound generating means is arranged to select a respective sound mode in dependence upon the control output.

9. A baby soother according to claim 8 in which the sound modes include at least one of a calming sound mode, a lullaby sound mode, and a volume reduction mode.

10. A baby soother according to any preceding claim in which the sound generating means is arranged to select a respective type of sound in dependence upon the control output.

11. A baby soother according to any preceding claim in which the sound bank stores pre-recorded tonal samples.

12. A baby soother according to any preceding claim in which the sound bank stores pre-recorded atonal sound samples.

13. A baby soother according to any preceding claim in which the sound bank stores user recorded sound samples.

14. A baby soother according to claim 11 in which the user recorded sound samples comprise at least one of a familiar voice, a familiar name and a familiar word.

15. A baby soother according to any preceding claim further comprising recording means for user recording of familiar sounds and for entering data representing the recorded sounds into the sound bank.

16. A baby soother according to any preceding claim in which the processor analyses the electrical signals to monitor at least one of the duration and the character of the crying.

17. A baby soother according to any preceding claim in which the processor generates a data analysis output based on the electrical signals, and compares the data analysis output with data in a reference bank storing data representing typical crying patterns in order to evaluate the character of the crying.

18. A baby soother according to any preceding claim further comprising means for generating an alarm signal in response to a predetermined control output from the processor indicating that the crying represents pain or hunger.

19. A baby soother according to any preceding claim in which the control output indicates intervals in the crying and in which the sound generating means generates the audio output to cause the output means to output sound in the intervals.

20. A baby soother according to any preceding claim in which the transducer comprises a microphone.

21. A baby soother according to any preceding claim in which the output means comprises a speaker.

22. A method of monitoring the crying by a baby comprising:
detecting crying by the baby and generating electrical signals representing the crying sound;
analysing the electrical signals to evaluate the crying;
providing a control output based on the evaluation;
storing audio data representing soothing sounds;
in response to the control output selecting audio data and providing an audio output; and
outputting the audio output as sound.
